# EUROPEAN PATENT APPLICATION

(11) **EP 2 134 078 A1**
(43) Date of publication of application: **16.12.2009**
(21) Application number: 07829039.2
(22) Date of filing: 02.10.2007
(51) Int. Cl.: H04N 5/225, A61B 1/04, G02B 23/24, G03B 17/02, H04N 5/335

(54) **IMAGING DEVICE, AND ENDOSCOPE**

(30) Priority: 16.01.2007 JP 2007007430
(71) Applicant: Olympus Medical Systems Corp., Tokyo 151-0072 (JP)
(72) Inventor: ISHII, Hiroshi, Tokyo 151-0072 (JP); ISHIDA, Yuya, Tokyo 151-0072 (JP); KUMEI, Kazuhiro, Tokyo 151-0072 (JP); AKIBA, Kazuyoshi, Tokyo 151-0072 (JP)
(74) Representative: von Hellfeld, Axel
(86) International application number: PCT/JP2007/069299
(87) International publication number: WO 2008/087771

(57) **Abstract**

An image pickup apparatus includes a solid-state image pickup device chip 32, an FPC 43 whose terminals are connected to the solid-state image pickup device chip 32, and a plurality of electronic components 70, 71, and 72 mounted on a front surface 43i of the FPC 43, wherein behind a back face 32h of the solid-state image pickup chip 32, the FPC 43 is disposed by being folded in such a way that the FPC 43 will provide mounting surfaces for the plurality of electronic components 70, 71, and 72 in a plurality of layers and that the plurality of electronic components 70, 71, and 72 will be superimposed via the FPC 43.

## Description

### Technical Field

The present invention relates to an image pickup apparatus and endoscope equipped with a solid-state image pickup device chip, a flexible printed circuit board connected to the solid-state image pickup device chip at one end, and multiple electronic components mounted at least on one side of the flexible printed circuit board.

### Background Art

In electronic endoscope apparatus which have come into widespread use in recent years, an image pickup apparatus which uses a solid-state image pickup device chip as image pickup means is disposed at a distal end of an insertion portion of an endoscope. In medical applications, for example, the insertion portion with the image pickup apparatus disposed is inserted in a body cavity to allow images of an area to be examined in the body cavity to be observed on a monitor.

Generally, principal part of the image pickup apparatus includes a solid-state image pickup device chip which has an image pickup surface, a cover glass which, being affixed to the image pickup surface, protects the image pickup surface, a TAB (Tape Automated Bonding) or other flexible printed circuit board (hereinafter referred to as an FPC) on which electronic components such as capacitors, resistors, and transistors are mounted, and a signal cable which transmits electrical signals of images of an area to be examined received from the solid-state image pickup device chip to external apparatus such as an image processing apparatus and monitor, where the FPC is connected at one end to a bonding portion of the solid-state image pickup device chip, and a lead wire connecting portion at the other end of the FPC is connected with lead wires of the signal cable.

To prevent the electronic components mounted on the FPC as well as other components in the lead wire connecting portion from contacting each other and downsize the image pickup apparatus, behind a back face opposite the image pickup surface of the solid-state image pickup device chip, the FPC conventionally is disposed in such a way as to be superimposed over a package unit whose principal part includes the cover glass, the solid-state image pickup chip, and bonding portion, when seen in planar view from the image pickup surface.

Specifically, the FPC is disposed, being inclined at a predetermined angle with respect to the back face of the solid-state image pickup device chip so that the FPC will be superimposed over the package unit when seen in planar view from the image pickup surface. That is, the FPC is disposed in such a way as substantially not to jut out from the package unit when seen in planar view from the image pickup surface.

However, downsizing of the package unit and increase in the number of electronic components mounted on the FPC pose a problem in that the FPC disposed behind the solid-state image pickup device chip juts out greatly from the package unit when seen in planar view from the image pickup surface.

In view of the above problem, Japanese Patent Application Laid-Open Publication No. 2000-210252 discloses a solid-state image pickup apparatus which can dispose an FPC so as not to jut out from a package unit when seen in planar view from an image pickup surface by providing plural bending lines on the FPC, bending the FPC into a box along the bending lines, housing plural electronic components and lead wire connecting portions efficiently in space of the FPC box even when the number of electronic components is increased or the package unit is downsized.

However, as shown in Japanese Patent Application Laid-Open Publication No. 2000-210252 described above, when the FPC is disposed behind the solid-state image pickup device chip by being bent into a box, there is a problem in that if the package unit is further downsized, it becomes difficult to lay out the plural electronic components and lead wire connecting portions in such a way as to prevent contact with one another and thereby avoid short-circuits in the space of the FPC box superimposed over the package unit when seen in planar view from the image pickup surface.

Also, Japanese Patent Application Laid-Open Publication No. 2000-210252 has a problem in that since lead wires of a signal cable are connected to the lead wire connecting portions before the FPC is bent into a box, lead wire connections tend to come off easily when the FPC is bent. On the other hand, if measures are taken to prevent the lead wire connections from coming off, it becomes difficult to bend the FPC.

Furthermore, downsizing of the package unit can be achieved by downsizing the electronic components mounted on the FPC. However, there is a problem in that if the electronic components are downsized, FPC is warped during bending of the FPC, causing the downsized electronic components to come easily off the FPC.

The present invention has been made in view of the above problems and has an object to provide an image pickup apparatus and endoscope having a configuration which makes it possible to downsize a package unit while preventing electronic components as well as lead wire connecting portions mounted on an FPC from contacting each other and improve ease of assembly by preventing electronic components from coming off the FPC.

### Disclosure of Invention

### Means for Solving the Problem

The present invention provides an image pickup apparatus including: a solid-state image pickup device chip; a flexible printed circuit board connected at one end to the solid-state image pickup device chip; and a plurality of electronic components mounted on one side of the flexible printed circuit board, wherein behind a back face opposite an image pickup surface of the solid-state image pickup chip, the flexible printed circuit board is disposed by being folded in such a way that the flexible printed circuit board will provide mounting surfaces for the plurality of electronic components in a plurality of layers and that the electronic components will be superimposed via the flexible printed circuit board.

Also, the present invention provides an endoscope which includes an image pickup apparatus, the image pickup apparatus including: a solid-state image pickup device chip; a flexible printed circuit board connected at one end to the solid-state image pickup device chip; and a plurality of electronic components mounted on one side of the flexible printed circuit board, wherein behind a back face opposite an image pickup surface of the solid-state image pickup chip, the flexible printed circuit board is disposed by being folded in such a way that the flexible printed circuit board will provide mounting surfaces for the plurality of electronic components in a plurality of layers and that the electronic components will be superimposed via the flexible printed circuit board.

### Brief Description of the Drawings

Fig. 1 is an external perspective view showing an endoscope apparatus which includes an endoscope and peripheral apparatus, where the endoscope includes an image pickup apparatus according to a first embodiment of the present invention;
Fig. 2 is a sectional view showing a configuration of an image pickup unit installed in a distal end portion of an insertion portion of the endoscope in Fig. 1;
Fig. 3 is a sectional view taken along line III-III in Fig. 2;
Fig. 4 is an exploded plan view showing an FPC of the image pickup apparatus in Fig. 3;
Fig. 5 is a partial plan view showing disposed positions of electronic components mounted in a first region of the FPC in Fig. 2, where the disposed positions are different from those in Fig. 4;
Fig. 6 is a diagram showing part of an image pickup apparatus according to a second embodiment;
Fig. 7 is an exploded plan view showing an FPC in Fig. 6;
Fig. 8 is a diagram showing part of an image pickup apparatus according to a third embodiment;
Fig. 9 is an exploded plan view showing an FPC in Fig. 8;
Fig. 10 is a diagram showing part of an image pickup apparatus according to a fourth embodiment;
Fig. 11 is an exploded plan view showing an FPC in Fig. 10;
Fig. 12 is a diagram showing part of an image pickup apparatus according to a fifth embodiment; and
Fig. 13 is an exploded plan view showing an FPC in Fig. 12.

### Best Mode for Carrying Out the Invention

Embodiments of the present invention will be described below with reference to the drawings. In the embodiments described below, it is assumed that an image pickup apparatus is installed in a distal end portion of an insertion portion of a medical endoscope.

### (First embodiment)

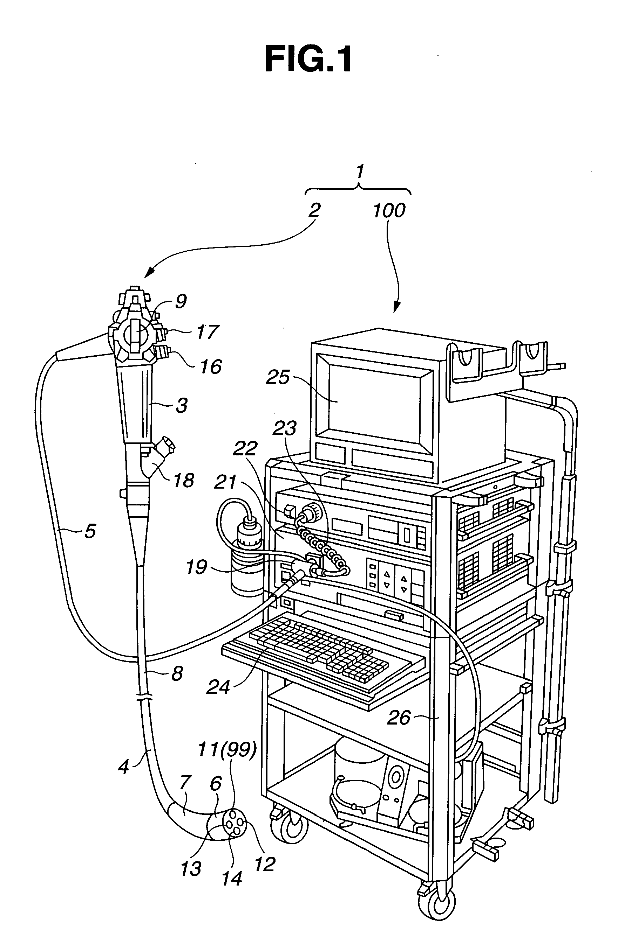

Fig. 1 is an external perspective view showing an endoscope apparatus which includes an endoscope and peripheral apparatus, where the endoscope includes an image pickup apparatus according to the present embodiment.

As shown in Fig. 1, the endoscope apparatus 1 includes the endoscope 2 and peripheral apparatus 100. Principal part of the endoscope 2 includes an operation portion 3, insertion portion 4, and universal cord 5.

Principal part of the peripheral apparatus 100 includes, a light source 21, video processor 22 which is an external apparatus, connection cable 23, keyboard 24, and monitor 25, all of which are placed on a rack 26. The endoscope 2 and peripheral apparatus 100 configured as described above are interconnected via a connector 19.

The operation portion 3 of the endoscope 2 is provided with a bending operation knob 9, air/water supply button 16, suction button 17, and treatment instrument insertion port 18.

The insertion portion 4 of the endoscope 2 includes a distal end portion 6, bending portion 7, and flexible tubular portion 8. The bending portion 7 is disposed between the distal end portion 6 and flexible tubular portion 8 and operated via the bending operation knob 9 installed in the operation portion 3.

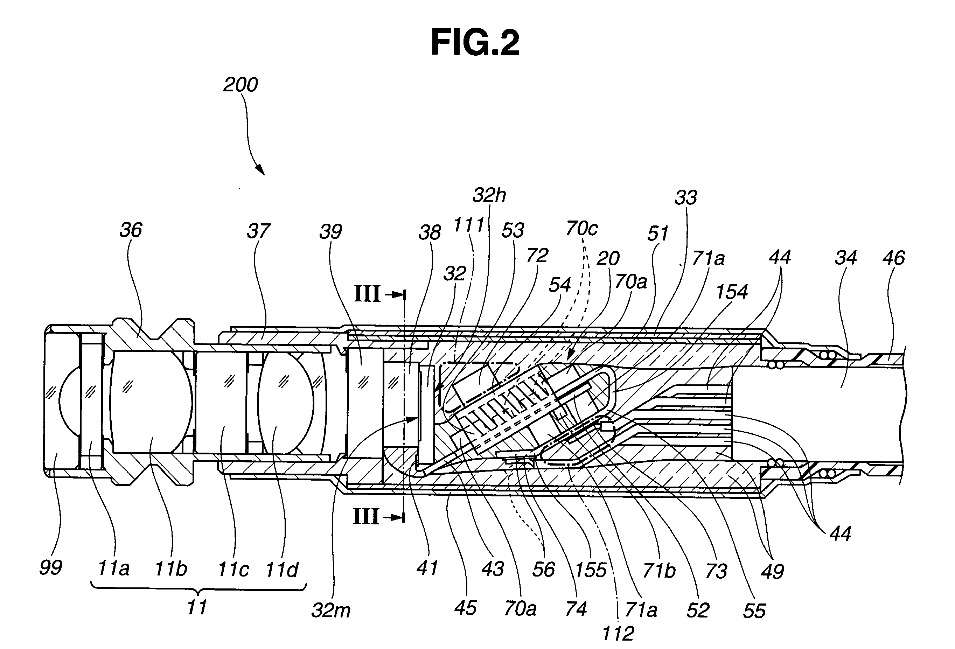

A cover glass 99 is disposed on a distal end face on a distal side in an insertion direction (hereinafter simply referred to as the distal side) of the distal end portion 6, where the cover glass 99 covers an objective lens 11a located on the distal side, in the insertion direction, of an objective lens group 11 of an image pickup unit 200 (described later) (see Fig. 2 for these components).

Also, in the distal end face on the distal side of the distal end portion 6, there are a nozzle 12, an illumination window 13, and a distal opening 14 of a treatment instrument passage (not shown), where the nozzle 12 is used to spray a fluid such as water or air onto a surface of the cover glass 99 and thereby clean the surface of the cover glass 99.

The distal end portion 6 incorporates an image pickup apparatus 20 of the image pickup unit 200 described later (see Fig. 2 for both).

The nozzle 12 selectively ejects gas and liquid when the air/water supply button 16 in the operation portion 3 is pressed. When the suction button 17 in the operation portion 3 is pressed, mucus and the like in a body cavity are selectively recovered through the distal opening 14 of the treatment instrument passage which extends in the insertion portion 4 from the distal opening 14 to the treatment instrument insertion port 18.

The connector 19 is installed at a tip of the universal cord 5 of the endoscope 2 and is connected to the light source 21 of the peripheral apparatus 100. The connector 19 is provided with a light guide ferrule (not shown) used to hold an end of a light guide (not shown), an electrical contact unit connected with an end of a signal cable 34 (described later; see Fig. 2), and the like. Furthermore, the connector 19 is connected with the connection cable 23 used to electrically connect the light source 21 to the video processor 22.

From the light guide ferrule (described above) on the connector 19, the light guide is inserted to a position near the illumination window 13 in the distal end portion 6 by passing through the universal cord 5, operation portion 3, and insertion portion 4 to transmit illumination light from the light source 21 to the illumination window 13 and thereby illuminate the body cavity widely through the illumination window 13.

The signal cable 34 is inserted from a solid-state image pickup device chip 32 (described later; see Fig. 2) of the image pickup apparatus 20 in the distal end portion 6 to the electrical contact unit (described above) of the connector 19 through the insertion portion 4, operation portion 3, and universal cord 5 to transmit electrical signals of images picked up by an image pickup surface 32m (described later; see Fig. 2) of the solid-state image pickup device chip 32 of the image pickup apparatus 20 to the video processor 22.

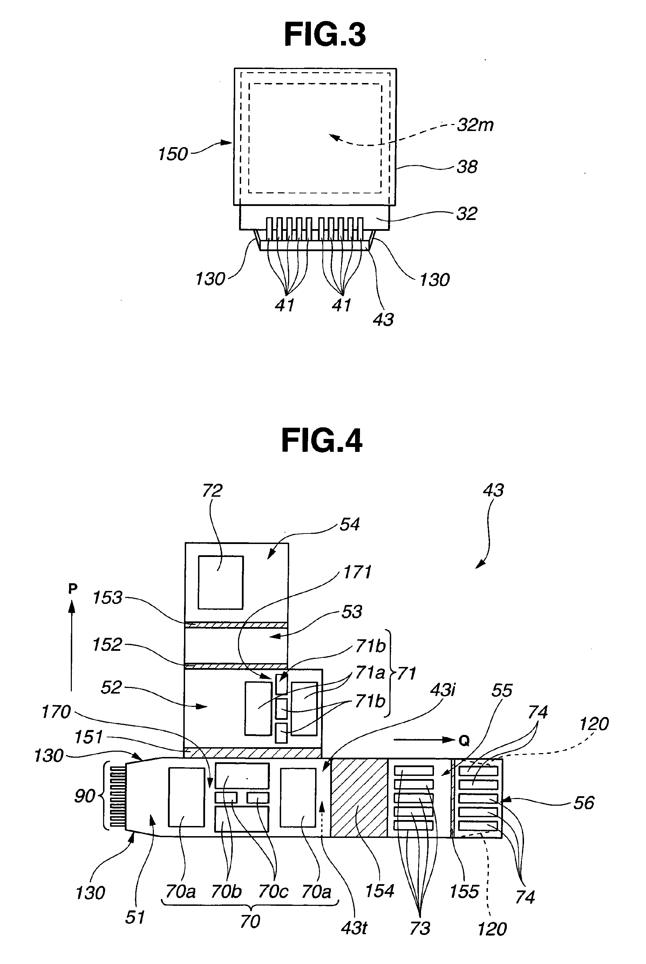

Next, a configuration of the image pickup apparatus 20 installed in the distal end portion 6 will be described with reference to Figs. 2 to 4. Fig. 2 is a sectional view showing a configuration of the image pickup unit installed in the distal end portion of the insertion portion of the endoscope in Fig. 1. Fig. 3 is a sectional view taken along line III-III in Fig. 2. Fig. 4 is an exploded plan view showing an FPC of the image pickup apparatus in Fig. 3.

As shown in Fig. 2, principal part of the image pickup unit 200 includes the objective lens group 11 made up of a plurality of objective lenses 11a to 11d, the cover glass 99 which covers the objective lens 11a placed on the distal side, in the insertion direction, of the objective lens group 11, a lens frame 36 which holds the objective lens group 11 and the cover glass 99, the image pickup apparatus 20, a device frame 37 which holds part of the image pickup apparatus 20, a shielding member 33, a heat-shrinkable tube 45, a protective tube 46, and thermoplastic resin 49.

An outer circumference on a rear side, in the insertion direction (hereinafter simply referred to as the rear side), of the lens frame 36 is fitted and fixed in an inner circumference on the distal side of the device frame 37. The distal side of the shielding member 33 is fixed to an outer circumference on the rear side of the device frame 37.

Furthermore, the heat-shrinkable tube 45 which covers outer circumferences of the device frame 37 and shielding member 33 is fixed to an outer circumference on the distal side of the device frame 37. The rear side of the heat-shrinkable tube 45 is fixed to an outer circumference on the distal side of the protective tube 46. The protective tube 46 covers an outer circumference of the signal cable 34 to protect the signal cable 34.

The image pickup apparatus 20 is disposed together with the thermoplastic resin 49 in an airtight space sealed by the shielding member 33 and heat-shrinkable tube 45, on the rear side, in the insertion direction (hereinafter simply referred to as the rear side), of the objective lens group 11.

Principal part of the image pickup apparatus 20 includes the solid-state image pickup device chip 32, a first cover glass 38, a second cover glass 39, an FPC 43, and the signal cable 34.

The first cover glass 38 is affixed to the image pickup surface 32m of the solid-state image pickup device chip 32 to protect the image pickup surface 32m. The second cover glass 39 larger in external dimensions than the first cover glass 38 is affixed to a distal end face of the first cover glass 38. Incidentally, an outer circumference of the second cover glass 39 is fixed to an inner circumference of the device frame 37.

Also, as shown in Fig. 3, the first cover glass 38, the solid-state image pickup device chip 32, and a bonding portion 41 form part of a package unit 150 of the image pickup apparatus 20, where the bonding portion 41 is a connecting portion of the solid-state image pickup device chip 32.

Behind a back face 32h of the solid-state image pickup device chip 32, with terminals 90 (described later; see Fig. 4) on one end of the FPC 43 being electrically connected to the bonding portion 41 of the solid-state image pickup device chip 32, for example, by soldering, and with lead wire connecting portions 73 and 74 (described later; see Fig. 4) on the other end of the FPC 43 being electrically connected with lead wires 44 of the signal cable 34 (see Fig. 3 for both), the FPC 43 is disposed obliquely at a predetermined angle with respect to the back face 32h by being folded into multiple layers in a region superimposed over the package unit 150 when the package unit 150 is seen in planar view from the image pickup surface 32m. A configuration of the FPC 43 will be described below with reference to Fig. 4.

As shown in Fig. 4, multiple electronic components 70 to 72 including capacitors, resistors, transistors and the like are mounted on one side, i.e., front side in Fig. 4 (hereinafter referred to as the front surface) 43i of the FPC 43, for example, by soldering. When the multiple electronic components 70 to 72 are mounted only on the front surface 43i in this way, it becomes easy to mount the electronic components 70 to 72 and possible to insulate the electronic components reliably from each other when the FPC 43 is folded into multiple layers.

Also, the lead wire connecting portions 73 and 74 are provided on the front surface 43i of the FPC 43 in order to be electrically connected with multiple lead wires 44 of the signal cable 34, for example, by soldering.

Specifically, as shown in Fig. 4, electronic components equal to or larger than a predetermined size, e.g., electronic components 70a and 70b equal to or larger than a known standard, 1005, are disposed circumferentially in a first region 51 on the front surface 43i of the FPC 43 when seen in planar view. Incidentally, the size of the electronic components 70a and 70b equal to or larger than a predetermined size is not limited to 1005, and may be 1608 alternatively.

Also, in the first region 51 on the front surface 43i of the FPC 43, electronic components smaller than the predetermined size, e.g., electronic components 70c smaller than the known standard, 1005, and compliant with a known standard, 0603, 0402, or the like, are disposed in a region 170 surrounded by the electronic components 70a and 70b equal to or larger than the known standard 1005 when seen in planar view.

Also, the terminals 90 connected to the bonding portion 41 of the solid-state image pickup device chip 32 are installed at the left end of the first region 51 in Fig. 4.

Furthermore, the first region 51 has a tapered portion 130, tapering down toward the end at which the terminals 90 are installed.

When, for example, an epoxy adhesive is applied to a predetermined thickness on the front surface 43i of the FPC 43 to fix the shape of the FPC 43 or reinforce the electronic components 70 to 72 mounted on the FPC 43, the tapered portion 130 serves to prevent the adhesive from running out of narrowed part of the first region 51 on the front surface 43i of the FPC 43.

Generally, when part near the terminals 90 narrows abruptly from the other part of the first region 51, if a thin coat of adhesive is applied to accommodate the narrower part, it is not possible to reinforce the electronic components mounted on the wider part sufficiently. On the other hand, if a thick coat of adhesive is applied to accommodate the wider part, the adhesive will run out of the narrower part. Consequently, there is a problem in that application thickness of the adhesive needs to be varied between the narrower part and wider part, requiring a complicated adhesive application operation.

However, with the FPC 43 of the image pickup apparatus 20 according to the present embodiment, since the tapered portion 130 is formed near the terminals 90 in the first region 51, even if a thick coat of adhesive is applied to accommodate the wider part, the tapered portion 130 whose shape changes gradually prevents the adhesive from running out of the narrower part. That is, even if the first region 51 of the FPC 43 contains the narrower part and wider part, the adhesive can be applied easily to a uniform thickness.

In the first region 51 of the FPC 43, the tapered portion 130, which narrows down toward the part where the terminals 90 are installed, allows the terminals 90 to be mounted on the bonding portion 41 of the solid-state image pickup device chip 32 by saving space as shown in Fig. 3. That is, the package unit 150 can be downsized.

Also, a second region 52 is provided above the first region 51 of the FPC 43 in Fig. 4 via a deformation portion 151. Electronic components equal to or larger than the predetermined size, e.g., electronic components 71a equal to or larger than the known standard, 1005, are disposed on the front surface 43i of the FPC 43 in the second region 52 in such a way that a longitudinal direction will be parallel to a folding direction P which is a first folding direction (described later). Incidentally, the size of the electronic components 71a equal to or larger than a predetermined size is not limited to 1005, and may be 1608 alternatively.

Furthermore, in the second region 52 on the front surface 43i of the FPC 43, electronic components smaller than the predetermined size, e.g., electronic components 71 b smaller than the known standard, 1005, and compliant with a known standard, 0603 or 0402 are disposed in a region 171 sandwiched by the electronic components 7 1 a when seen in planar view.

That is, the electronic components 71a equal to or larger than the predetermined size are disposed in such a way as to sandwich the electronic components 71b smaller than the predetermined size in a direction orthogonal to the folding direction P, when seen in planar view.

Also, a third region 53 is provided above the second region 52 of the FPC 43 in Fig. 4 via a deformation portion 152. Furthermore, a fourth region 54 is provided above the third region 53 of the FPC 43 in Fig. 4 via a deformation portion 153. Electronic components equal to or larger than the predetermined size, e.g., electronic components 72 equal to or larger than the known standard, 1005, are disposed on the front surface 43i of the FPC 43 in the fourth region 54.

Also, a fifth region 55 is provided on the right side of the first region 51 of the FPC 43 in Fig. 4 via a deformation portion 154 and the lead wire connecting portion 73 is provided on the front surface 43i of the FPC 43 in the fifth region 55.

Furthermore, a sixth region 56 is provided on the right side of the fifth region 55 of the FPC 43 in Fig. 4 via a deformation portion 155 and the lead wire connecting portion 74 is provided on the front surface 43i of the FPC 43 in the sixth region 56.

A tapered portion 120 may be formed at the right end of the sixth region 56 in Fig. 4 as indicated by dotted lines. The tapered portion 120 is intended to prevent ends of the sixth region 56 of the FPC 43 from jutting out from the package unit 150 without folding the sixth region 56 when seen in planar view from the image pickup surface 32m.

The FPC 43 configured as described above is disposed obliquely at a predetermined angle with respect to the back face 32h in a region superimposed over the package unit 150 when seen in planar view from the image pickup surface 32m. Also, as shown in Fig. 2, the FPC 43 is disposed, being folded in such a way that the front surface 43i of the FPC 43 will provide mounting surfaces for the electronic components 70 to 72 in three layers and that the electronic components 70 to 72 will be superimposed via the FPC 43.

If the FPC 43 is folded in a region superimposed over the package unit 150 when seen in planar view in such a way that the front surface 43i of the FPC 43 will provide mounting surfaces for the electronic components 70 to 72 in one or two layers, a region 111 surrounded by a dash-and-dot line in Fig. 2 becomes dead space. However, when the FPC 43 is folded to provide mounting surfaces for the electronic components 70 to 72 on the front surface 43i of the FPC 43 in three layers, as with the present embodiment, the electronic components 72 can be disposed in the region 111. Thus, the region 111 can be used effectively to dispose the FPC 43 behind the back face 32h of the solid-state image pickup device chip 32. This allows high-density mounting of electronic components on the FPC 43.

Furthermore, as shown in Fig. 2, the lead wire connecting portions 73 and 74 are disposed in a region different from the regions in which the electronic components 70 to 72 are mounted, specifically, in a region below the regions in which the electronic components 70 to 72 are mounted. This makes it possible to downsize mounting space of the FPC 43.

Now, a method for folding the FPC 43 configured as shown in Fig. 4 into a shape shown in Fig. 2 will be described below.

Specifically, the terminals 90 in the first region 51 are electrically connected to the bonding portion 41 of the solid-state image pickup device chip 32 by soldering or the like. Then, as shown in Fig. 2, the first region 51 is bent obliquely upward in Fig. 2 so as to be at a predetermined angle to the back face 32h of the solid-state image pickup device chip 32. Consequently, the electronic components 70 mounted on the front surface 43i of the FPC 43 in the first region 51 are oriented obliquely upward in Fig. 2.

Next, the second region 52 is folded below the first region 51. Specifically, the second region 52 is folded in the folding direction P over the first region 51 by bending the deformation portion 151 so that a rear surface 43t on the other side of the FPC 43 in the second region 52 will face the rear surface 43t of the FPC 43 in the first region 51. Consequently, the electronic components 71 mounted on the front surface 43i of the FPC 43 in the second region 52 are oriented obliquely downward in Fig. 2.

Thus, the electronic components 71 are disposed out of direct contact with the electronic components 70. In other words, the electronic components 71 are superimposed over the electronic components 70 via the first region 51 and second region 52.

Next, by deforming the deformation portion 152, the third region 53 is bent in the folding direction P so as to be approximately perpendicular to the front surface 43i of the FPC 43 in the first region 51.

Next, the fourth region 54 is folded over the first region 51. Specifically, the fourth region 54 is folded in the folding direction P by bending the deformation portion 153 so that the rear surface 43t of the FPC 43 in the fourth region 54 will face the front surface 43i of the FPC 43 in the first region 51.

Consequently, the electronic components 72 mounted on the front surface 43i of the FPC 43 in the fourth region 54 are oriented obliquely upward in Fig. 2. Thus, the electronic components 72 are disposed out of direct contact with the electronic components 70 and 71. In other words, the electronic components 72 are superimposed over the electronic components 70 via the third region 53 and fourth region 54. Also, the electronic components 72 are superimposed over the electronic components 71 via the first region 51 to the fourth region 54.

Next, the fifth region 55 is folded below the second region 52. Specifically, the fifth region 55 is folded in a folding direction Q which is a second folding direction by bending the deformation portion 154 so that the rear surface 43t of the FPC 43 in the fifth region 55 will face the front surface 43i of the FPC 43 in the second region 52.

Consequently, the lead wire connecting portion 73 on the front surface 43i of the FPC 43 in the fifth region 55 is oriented obliquely downward in Fig. 2. Thus, the lead wire connecting portion 73 is disposed out of direct contact with the electronic components 71. In other words, the lead wire connecting portion 73 are superimposed over the electronic components 71 via the fifth region 55.

Next, the sixth region 56 is bent upward in Fig. 2 in the folding direction Q by deforming the deformation portion 155. Consequently, the sixth region 56 is disposed without jutting out partially from the package unit 150 when seen in planar view from the image pickup surface 32m. The lead wire connecting portion 74 on the front surface 43i of the FPC 43 in the sixth region 56 is oriented obliquely downward in Fig. 2.

Incidentally, the fifth region 55 and sixth region 56, which are placed in the bottom layer of the folded FPC 43, are located in regions different from the regions in which the electronic components 70 to 72 are mounted.

When the tapered portion 120 is formed in the sixth region 56, even if the sixth region 56 is not bent as indicated by a dotted line in Fig. 2, the sixth region 56 does not jut out partially from the package unit 150 when seen in planar view from the image pickup surface 32m.

Subsequently, to fix the shape of the folded FPC 43 and reinforce the electronic components 70 to 73, an adhesive is applied to a predetermined thickness on the FPC 43. The tapered portion 130 prevents the adhesive from running out of the narrowed part near the terminals 90 in the first region 51, as described above.

Finally, the lead wire connecting portions 73 and 74 provided on the front surface 43i of the FPC 43 in the fifth region 55 and sixth region 56 are electrically connected with multiple lead wires 44 of the signal cable 34, for example, by soldering.

The lead wire connecting portions 73 and 74, which are located in regions different from the regions in which the electronic components 70 to 72 are mounted, specifically, in the bottom layer of the folded FPC 43, and oriented obliquely downward in Fig. 2, are to be easily connected with the multiple lead wires 44 of the signal cable 34. This makes it possible to downsize the mounting space of the FPC 43.

If the FPC 43 is folded in a region superimposed over the package unit 150 when seen in planar view in such a way that the front surface 43i of the FPC 43 will provide mounting surfaces for the electronic components 70 to 72 in one or two layers, a region 112 surrounded by a dash-and-dot line in Fig. 2 becomes dead space. However, according to the present embodiment, since the FPC 43 is folded to provide mounting surfaces for the electronic components 70 to 72 on the front surface 43i of the FPC 43 in three layers and consequently the lead wire connecting portions 73 and 74 are disposed in the region 112, making the region 112 available for use to connect with the lead wires 44, the region 112 can be used effectively to dispose the FPC 43 behind the back face 32h of the solid-state image pickup device chip 32.

Consequently, the FPC 43 is disposed behind the solid-state image pickup chip 43 in the shape described above.

In this way, according to the present embodiment, behind the solid-state image pickup device chip 32, the FPC 43 is disposed obliquely at a predetermined angle with respect to the back face 32h, in a region superimposed over the package unit 150 when seen in planar view from the image pickup surface 32m.

Also, as described above, the FPC 43 is disposed, being folded in such a way that the front surface 43i of the FPC 43 will provide mounting surfaces for the electronic components 70 to 72 in three layers and that the electronic components 70 to 72 will be superimposed via the regions 51 to 55 of the FPC 43.

This makes it possible to dispose the FPC 43 reliably behind the solid-state image pickup device chip 32 without jutting out from the package unit 150 when seen in planar view from the image pickup surface 32m. Also, the electronic components 70 to 72 as well as the lead wire connecting portions 73 and 74, which are superimposed over each other via the regions 51 to 55 of the FPC 43, are kept out of contact from each other.

Also, according to the present embodiment, as described above, in the first region 51 on the front surface 43i of the FPC 43, electronic components 71c smaller than a predetermined size are disposed in a region 170 surrounded by the electronic components 70a and 70b equal to or larger than the predetermined size, when seen in planar view.

Consequently, when the second region 52 to the fourth region 54 are folded in the folding direction P over the first region 51, even if the first region 51 warps due to an external force acting on the first region 51 and pulling the first region 51 in the folding direction P, strength, i.e., rigidity, of the first region 51 in the folding direction P is increased by the electronic components 70a which are equal to or larger than the predetermined size and mounted in the first region 51 in such a way as to sandwich the electronic components 70c smaller than the predetermined size in the direction orthogonal to the folding direction P. This reduces the tendency of the electronic components 70c smaller than the predetermined size to separate from the FPC 43 due to warping of the first region 51 and thus improves ease of assembly of the image pickup apparatus 20.

Also, when the fifth region 55 and sixth region 56 are folded over the first region 51 in the folding direction Q, even if the first region 51 warps due to an external force acting on the first region 51 and pulling the first region 51 in the folding direction Q, strength, i.e., rigidity, of the first region 51 in the folding direction Q is increased by the electronic components 70b which are equal to or larger than the predetermined size and mounted in the first region 51 in such a way as to sandwich the electronic components 70c smaller than the predetermined size in a direction orthogonal to the folding direction Q. This reduces the tendency of the electronic components 70c smaller than the predetermined size to separate from the FPC 43 due to warping of the first region 51 and thus improves ease of assembly of the image pickup apparatus 20.

Furthermore, according to the present embodiment, as described above, in the second region 52 on the front surface 43i of the FPC 43, the electronic components 71a equal to or larger than the predetermined size are disposed in such a way as to sandwich the electronic components 71b smaller than the predetermined size in the direction orthogonal to the folding direction P, when seen in planar view.

Consequently, when the third region 53 and fourth region 54 are folded in the folding direction P over the second region 52, even if the second region 52 warps due to an external force acting on the second region 52 and pulling the second region 52 in the folding direction P, strength, i.e., rigidity, of the second region 52 in the folding direction P is increased by the electronic components 71a which are equal to or larger than the predetermined size and mounted in the second region 52 in such a way as to sandwich the electronic components 71 b smaller than the predetermined size in the direction orthogonal to the folding direction P. This reduces the tendency of the electronic components 71b smaller than the predetermined size to separate from the FPC 43 due to warping of the second region 52 and thus improves ease of assembly of the image pickup apparatus 20.

Thus, the present embodiment provides the image pickup apparatus 20 having a configuration which makes it possible to downsize the package unit 150 while preventing the electronic components 70 to 72 as well as lead wire connecting portions 73 and 74 mounted on the FPC 54 from contacting each other and improve ease of assembly by preventing the electronic components 70 to 72 from coming off the FPC 43.

Now, a variation will be shown below. According to the present embodiment, as described above, in the second region 52 on the front surface 43i of the FPC 43, the electronic components 7 1 a equal to or larger than the predetermined size are disposed in such a way as to sandwich the electronic components 71 b smaller than the predetermined size in the direction orthogonal to the folding direction P, when seen in planar view.

This is not restrictive. As long as a large area can be secured for the second region 52, the electronic components 71a equal to or larger than the predetermined size may be disposed in such a way as to surround the electronic components 71b smaller than the predetermined size, when seen in planar view as in the case of the electronic components 70. This configuration also reduces the tendency of the electronic components 71 b smaller than the predetermined size to separate from the FPC 43 due to warping of the second region 52.

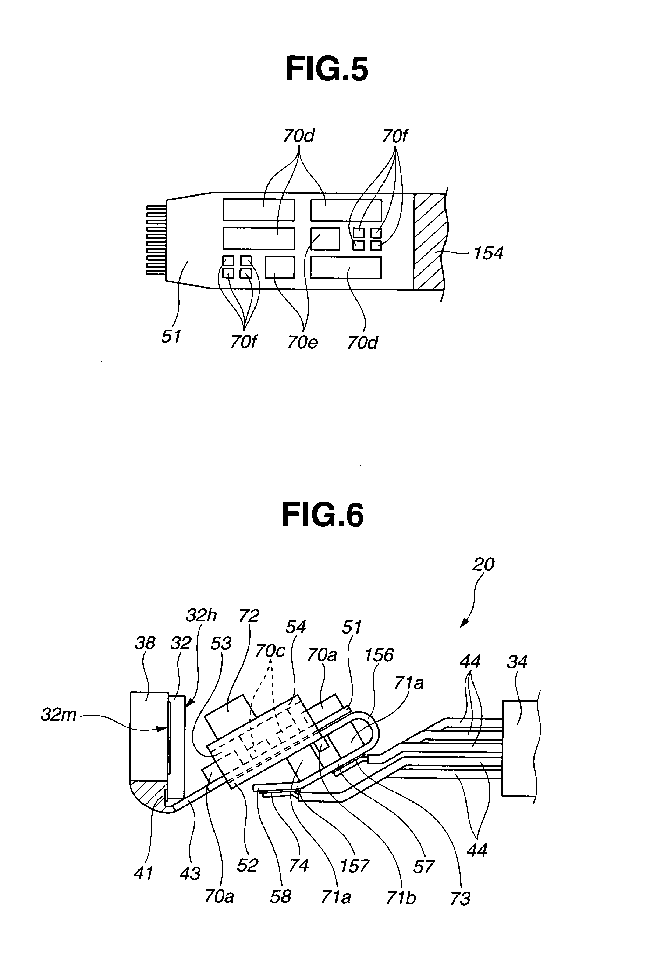

Another variation will be described below with reference to Fig. 5. Fig. 5 is a partial plan view showing disposed positions of electronic components mounted in the first region of the FPC in Fig. 2, where the disposed positions are different from those in Fig. 4.

According to the present embodiment, as described above, in the first region 51 on the front surface 43i of the FPC 43, the electronic components 70c smaller than the predetermined size are disposed in the region 170 surrounded by the electronic components 70a and 70b equal to or larger than the predetermined size, when seen in planar view.

This is not restrictive. As shown in Fig. 5, for example, electronic components smaller than a predetermined size, e.g., electronic components 70f smaller than the known standard, 1005, and compliant with a known standard, 0603 or 0402, may be disposed near electronic components 70d and 70e equal to or larger than the known standard 1005.

With this configuration, even if the first region 51 warps due to external forces acting on the first region 51 in the folding directions P and Q, strength, i.e., rigidity, of the first region 51 in the folding directions P and Q is increased by the electronic components 70d and 70e equal to or larger than the predetermined size, reducing the tendency of the electronic components 70f smaller than the predetermined size to separate from the FPC 43.

### (Second embodiment)

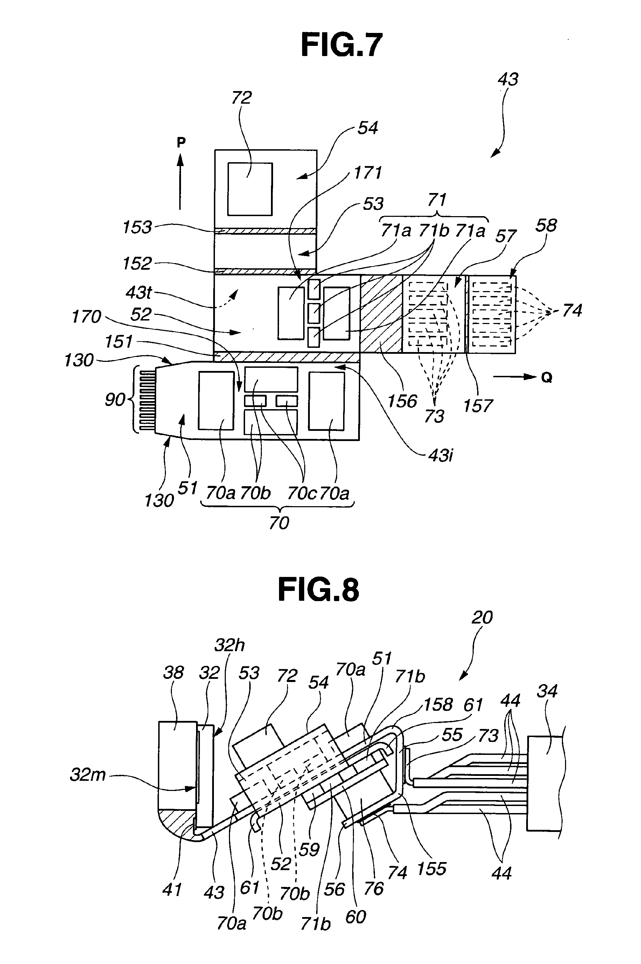

Fig. 6 is a diagram showing part of an image pickup apparatus according to the present embodiment. Fig. 7 is an exploded plan view showing an FPC in Fig. 6.

A configuration of the image pickup apparatus according to the second embodiment differs from the image pickup apparatus according to the first embodiment shown in Figs. 1 to 4 in that lead wire connecting portions are installed on a rear side of the FPC. Only the difference will be described. On the other hand, the same components as the first embodiment will be denoted by the same reference numerals as the corresponding components in the first embodiment, and description thereof will be omitted.

As shown in Fig. 7, a seventh region 57 is provided on the right side of the second region 52 of the FPC 43 in Fig. 7 via a deformation portion 156 and the lead wire connecting portion 73 is provided on the rear surface 43t of the FPC 43 in the seventh region 57. The deformation portion 156 has a smaller surface area than the deformation portion 154 according to the first embodiment described above.

Furthermore, an eighth region 58 is provided on the right side of the seventh region 57 of the FPC 43 in Fig. 7 via a deformation portion 157 and the lead wire connecting portion 74 is provided on the rear surface 43t of the FPC 43 in the eighth region 58. A tapered portion 120 may be formed also in the eighth region 58 as in the case of the first embodiment described above.

The FPC 43 configured as described above is disposed obliquely at a predetermined angle with respect to the back face 32h in a region superimposed over the package unit 150 when seen in planar view from the image pickup surface 32m. Also, as shown in Fig. 6, the FPC 43 is disposed, being folded in such a way that the front surface 43i of the FPC 43 will provide mounting surfaces for the electronic components 70 to 72 in three layers and that the electronic components 70 to 72 will be superimposed via the FPC 43.

Furthermore, the lead wire connecting portions 73 and 74 are disposed in regions different from the regions in which the electronic components 70 to 72 are mounted, specifically, in the bottom layer of the folded FPC 43 as shown in Fig. 6.

Now, a method for folding the FPC 43 configured as shown in Fig. 7 into a shape shown in Fig. 6 will be described below.

Specifically, the first region 51 to the fourth region 54 are folded in the folding direction P as in the case of the first embodiment described above.

Next, the seventh region 57 is folded below the second region 52 by bending the deformation portion 156. Specifically, the seventh region 57 is folded in the folding direction Q so that the front surface 43i of the FPC 43 in the seventh region 57 will face the front surface 43i of the FPC 43 in the second region 52.

According to the present embodiment, since no region is superimposed between the second region 52 and seventh region 57 as shown in Fig. 6, bendable width of the deformation portion 156 can be made smaller than in the first embodiment described above.

Consequently, the lead wire connecting portion 73 provided on the rear surface 43t of the FPC 43 in the seventh region 57 is oriented obliquely downward in Fig. 6. Thus, the lead wire connecting portion 73 is disposed out of direct contact with the electronic components 71. In other words, the lead wire connecting portion 73 is superimposed over the electronic components 71 via the seventh region 57.

Next, the eighth region 58 is bent upward in Fig. 6 in the folding direction Q by deforming the deformation portion 157. Consequently, the eighth region 58 is disposed without jutting out partially from the package unit 150 when seen in planar view from the image pickup surface 32m.

Incidentally, the seventh region 57 and eighth region 58, which are placed in the bottom layer of the folded FPC 43, are located in regions different from the regions in which the electronic components 70 to 72 are mounted.

Subsequent processes are the same as those of the first embodiment, and thus description thereof will be omitted.

Consequently, the FPC 43 is disposed behind the solid-state image pickup chip 43 in the shape described above.

In this way, according to the present embodiment, the lead wire connecting portions 73 and 74 are provided on the rear surface 43t of the FPC 43 in the seventh region 57 and eighth region 58 located on the right side of the second region 52 in Fig. 6.

Thus, the present embodiment provides the same advantages as those of the first embodiment. Besides, since the seventh region 57 with the lead wire connecting portion 73 mounted is installed immediately below the second region 52, bendable width of the deformation portion 156 can be made smaller than in the first embodiment. That is, the FPC 43 can be disposed behind the solid-state image pickup device chip 32 more compactly than in the first embodiment described above.

### (Third embodiment)

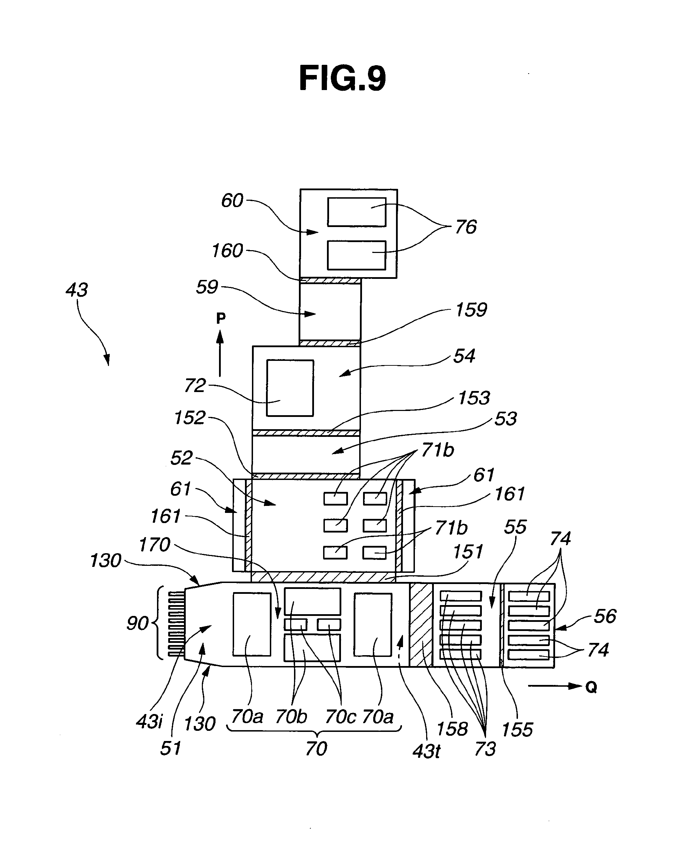

Fig. 8 is a diagram showing part of an image pickup apparatus according to the present embodiment and Fig. 9 is an exploded plan view showing an FPC in Fig. 8.

A configuration of the image pickup apparatus according to the third embodiment differs from the image pickup apparatus according to the first embodiment shown in Figs. 1 to 4 in that mounting surfaces for the electronic components are provided on the front surface of the FPC in four layers and that a mounting strength enhancement portion is provided in the second region. Only the differences will be described. On the other hand, the same components as the first embodiment will be denoted by the same reference numerals as the corresponding components in the first embodiment, and description thereof will be omitted.

As shown in Fig. 9, a ninth region 59 is provided above the fourth region 54 of the FPC 43 in Fig. 9 via a deformation portion 159. Also, a tenth region 60 is provided above the ninth region 59 in Fig. 9 via a deformation portion 160.

Electronic components equal to or larger than the predetermined size, e.g., electronic components 76 equal to or larger than the known standard, 1005, are disposed on the front surface 43i of the FPC 43 in the tenth region 60.

Also, a mounting strength enhancement portion 61 is located via a deformation portion 161 on each end of the second region 52 in the direction orthogonal to the folding direction P to enhance mounting strength in the folding direction P in the second region 52. Bending the mounting strength enhancement portions 61 toward the second region 52 increases the mounting strength of the electronic components 71 b smaller than the predetermined size in the folding direction P in the second region 52.

Furthermore, on the front surface 43i of the FPC 43 in the second region 52, the electronic components 71 b smaller than the predetermined size are disposed near a mounting strength enhancement portion 61.

According to the present embodiment, as in the case of the first embodiment described above, a tapered portion 120 may be provided in the sixth region 56.

The FPC 43 configured as described above is disposed obliquely at a predetermined angle with respect to the back face 32h in a region superimposed over the package unit 150 when seen in planar view from the image pickup surface 32m.

Also, as shown in Fig. 8, the FPC 43 is disposed, being folded in such a way that the front surface 43i of the FPC 43 will provide mounting surfaces for the electronic components 70 to 72 and 76 in four layers and that the electronic components 70 to 72 and 76 will be superimposed via the FPC 43.

Furthermore, as shown in Fig. 8, on the FPC 43, the lead wire connecting portions 73 and 74 are disposed in regions different from the regions in which the electronic components 70 to 72 and 76 are mounted.

Now, a method for folding the FPC 43 configured as shown in Fig. 9 into a shape shown in Fig. 8 will be described below.

Specifically, first, the first region 51 to the fourth region 54 are folded in the folding direction P as in the case of the first embodiment described above. Also, by deforming the deformation portion 161, the mounting strength enhancement portions 61 in the second region 52 are bent downward in Fig. 8 so as to be substantially perpendicular to the front surface 43i of the FPC 43 in the second region 52. This increases the mounting strength in the folding direction P in the second region 52.

Next, by deforming the deformation portion 159, the ninth region 59 is bent in the folding direction P so as to be perpendicular to the rear surface 43t of the FPC 43 in the fourth region 54. Then, the tenth region 60 is folded below the second region 52. Specifically, by bending the deformation portion 160, the tenth region 60 is folded in the folding direction P so that the rear surface 43t of the FPC 43 will face the front surface 43i of the FPC 43 in the second region 52.

Consequently, electronic components 76 mounted on the front surface 43i of the FPC 43 in the tenth region 60 are oriented obliquely downward in Fig. 8.

Thus, the electronic components 76 are disposed out of direct contact with the electronic components 71. In other words, the electronic components 76 are superimposed over the electronic components 71 via the ninth region 59 and tenth region 60.

Finally, by bending the deformation portion 158, the fifth region 55 is bent in the folding direction Q so that the rear surface 43t of the FPC 43 in the fifth region 55 will be substantially parallel to the back face 32h of the solid-state image pickup device chip 32. Then, the sixth region 56 is folded below the tenth region 60. Specifically, by bending the deformation portion 155, the sixth region 56 is folded in the folding direction Q so that the rear surface 43t of the FPC 43 in the sixth region 56 will face the front surface 43i of the FPC 43 in the tenth region 60.

Subsequent processes are the same as those of the first embodiment, and thus description thereof will be omitted.

Consequently, the FPC 43 is disposed behind the solid-state image pickup chip 43 in the shape and positions described above.

In this way, according to the present embodiment, the FPC 43 is folded in such a way that the front surface 43i of the FPC 43 will provide mounting surfaces for the electronic components 70 to 72 and 76 in four layers, and this configuration provides advantages similar to the advantages of the image pickup apparatus 20 according to the first embodiment in which the FPC 43 is folded in such a way that the front surface 43i of the FPC 43 will provide mounting surfaces for the electronic components 70 to 72 in three layers.

Also, according to the present embodiment, as described above, the mounting strength enhancement portion 61 is located via the deformation portion 161 on each end of the second region 52 in the direction orthogonal to the folding direction P to enhance mounting strength in the folding direction P in the second region 52. Furthermore, as described above, on the front surface 43i of the FPC 43 in the second region 52, the electronic components 71 b smaller than the predetermined size are disposed near a mounting strength enhancement portion 61.

Consequently, on the front surface 43i of the FPC 43 in the second region 52, even if the electronic components 71 b smaller than the predetermined size are not disposed in such a way as to be sandwiched, when seen in planar view, by the electronic components 71a equal to or larger than the predetermined size in the direction orthogonal to the folding direction when seen in planar view as in the case of the first embodiment described above, by simply disposing the electronic components 71 b smaller than the predetermined size near the mounting strength enhancement portions 61, it is possible to reliably prevent the electronic components 71b smaller than the predetermined size from coming off the front surface 43i of the FPC 43 due to warping of the second region 52 caused by an external force acting in the folding direction P on the second region 52.

### (Fourth embodiment)

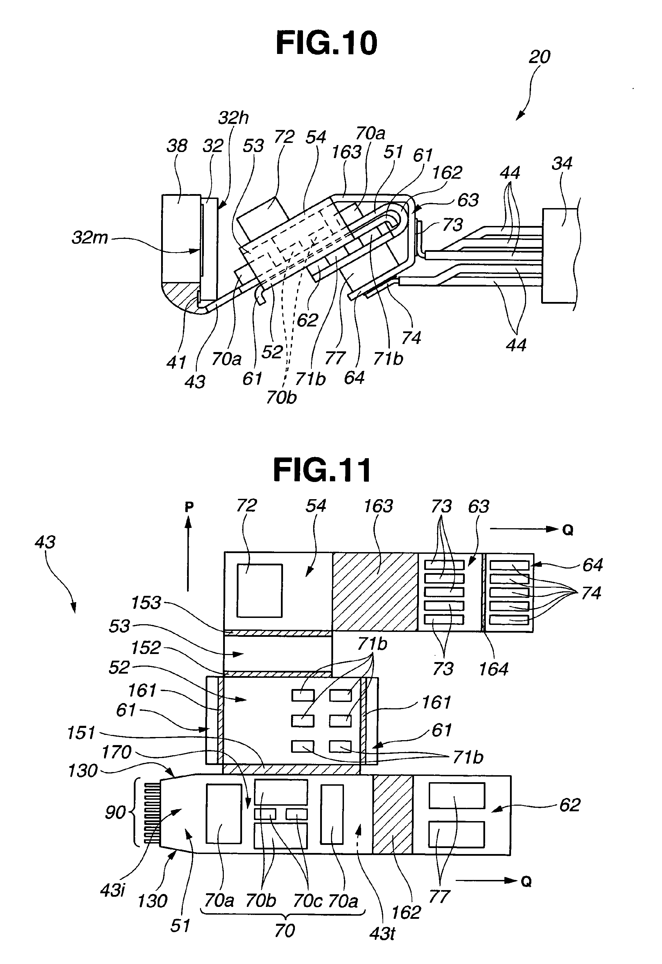

Fig. 10 is a diagram showing part of an image pickup apparatus according to the present embodiment and Fig. 11 is an exploded plan view showing an FPC in Fig. 10.

A configuration of the image pickup apparatus according to the fourth embodiment differs from the image pickup apparatus described above according to the third embodiment shown in Figs. 8 and 9 in that part of electronic components is also installed in an FPC region folded in the folding direction Q. Only the differences will be described. On the other hand, the same components as the third embodiment will be denoted by the same reference numerals as the corresponding components in the third embodiment, and description thereof will be omitted.

As shown in Fig. 11, an eleventh region 62 is provided on the right side of the first region 51 of the FPC 43 in Fig. 11 via a deformation portion 162, and electronic components equal to or larger than the predetermined size, e.g., electronic components 77 equal to or larger than the known standard, 1005, are disposed on the front surface 43i of the FPC 43 in the eleventh region 62.

A twelfth region 63 is provided on the right side of the fourth region 54 of the FPC 43 in Fig. 11 via a deformation portion 163, and the lead wire connecting portion 73 is installed on the front surface 43i of the FPC 43 in the twelfth region 63.

Also, a thirteenth region 64 is provided on the right side of the twelfth region 63 of the FPC 43 via a deformation portion 164 and the lead wire connecting portion 74 is installed on the front surface 43i of the FPC 43 in the thirteenth region 64.

A tapered portion 120 may be provided in the thirteenth region 64 as in the case of the first embodiment.

The FPC 43 configured as described above is disposed obliquely at a predetermined angle with respect to the back face 32h in a region superimposed over the package unit 150 when seen in planar view from the image pickup surface 32m.

Also, as shown in Fig. 10, the FPC 43 is disposed, being folded in such a way that the front surface 43i of the FPC 43 will provide mounting surfaces for the electronic components 70 to 72 and 77 in four layers and that the electronic components 70 to 72 and 77 will be superimposed via the FPC 43.

Furthermore, as shown in Fig. 10, on the FPC 43, the lead wire connecting portions 73 and 74 are disposed in regions different from the regions in which the electronic components 70 to 72 and 77 are mounted, specifically, in the bottom layer.

Now, a method for folding the FPC 43 configured as shown in Fig. 11 into a shape shown in Fig. 10 will be described below.

Specifically, the first region 51 to the fourth region 54 are folded in the folding direction P as in the case of the first embodiment described above.

Next, the eleventh region 62 is folded below the second region 52. Specifically, by bending the deformation portion 162, the eleventh region 62 is folded in the folding direction Q so that the rear surface 43t of the FPC 43 in the eleventh region 62 will face the front surface 43i of the FPC 43 in the second region 52.

Consequently, the electronic components 77 mounted on the front surface 43i of the FPC 43 in the eleventh region 62 are oriented obliquely downward in Fig. 10.

Thus, the electronic components 77 are disposed out of direct contact with the electronic components 71. In other words, the electronic components 77 are superimposed over the electronic components 71 via the eleventh region 62.

Finally, by bending the deformation portion 163, the twelfth region 63 is folded in the folding direction Q so that the rear surface 43t of the FPC 43 will be substantially parallel to the back face 32h of the solid-state image pickup device chip 32. Then, the thirteenth region 64 is folded below the eleventh region 62. Specifically, by bending the deformation portion 164, the thirteenth region 64 is folded in the folding direction Q so that the rear surface 43t of the FPC 43 in the thirteenth region 64 will face the front surface 43i of the FPC 43 in the eleventh region 62.

Subsequent processes are the same as those of the first embodiment, and thus description thereof will be omitted.

Consequently, the FPC 43 is disposed behind the solid-state image pickup chip 43 in the shape and positions described above.

In this way, according to the present embodiment, the FPC 43 is folded in such a way that the front surface 43i of the FPC 43 will provide mounting surfaces for the electronic components 70 to 72 and 77 in four layers, and the folding direction Q of the eleventh region 62 in which the electronic components 77 are mounted is different from the folding direction P of the second region 52 and fourth region 54 in which the other electronic components 71 and 72 are mounted.

This configuration also provides advantages similar to the advantages of the third embodiment. Besides, compared to the third embodiment described above, since the number of times of folding in the folding direction P is reduced by one, width of the FPC 43 as seen in planar view from the image pickup surface 32m can be made smaller than in the third embodiment.

### (Fifth embodiment)

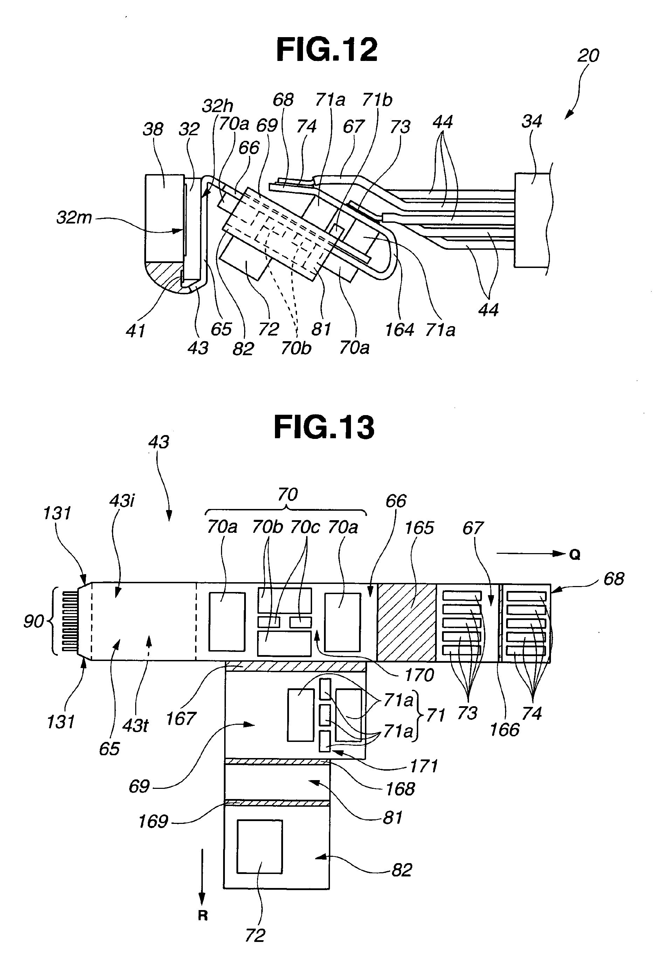

Fig. 12 is a diagram showing part of an image pickup apparatus according to the present embodiment and Fig. 13 is an exploded plan view showing an FPC in Fig. 12.

A configuration of the image pickup apparatus according to the fifth embodiment differs from the image pickup apparatus according to the first embodiment shown in Figs. 1 to 4 in the folding direction of the regions in the FPC in which the electronic components are mounted. Only the differences will be described. On the other hand, the same components as the first embodiment will be denoted by the same reference numerals as the corresponding components in the first embodiment, and description thereof will be omitted.

As shown in Fig. 13, a fourteenth region 65 on the rear surface 43t of the FPC 43 constitutes a bonding surface bonded to the back face of the solid-state image pickup device chip 32 when the FPC 43 is folded. According to the present embodiment, a tapered portion 131 similar in function to the tapered portion 130 described above is formed near the terminals 90 in the fourteenth region 65.

Also, a fifteenth region 66 is located on the right side of the fourteenth region 65 of the FPC 43 in Fig. 13. The fifteenth region 66 is similar in configuration to the first region 55. Furthermore, a sixteenth region 67 is located on the right side of the fifteenth region 66 of the FPC 43 in Fig. 13 via a deformation portion 165 and a seventeenth region 68 is located on the right side of the sixteenth region 67 of the FPC 43 in Fig. 13 via a deformation portion 166. The sixteenth region 67 and seventeenth region 68 are similar in configuration to the fifth region 55 and sixth region 56. A tapered portion 120 may be provided also in the seventeenth region 68 as in the case of the first embodiment.

Also, an eighteenth region 69 is located below the fifteenth region 66 in Fig. 13 via a deformation portion 167, a nineteenth region 81 is located below the eighteenth region 69 in Fig. 13 via a deformation portion 168, and a twentieth region 82 is provided below the nineteenth region 81 in Fig. 13 via a deformation portion 169.

Incidentally, the eighteenth region 69, nineteenth region 81, and twentieth region 82 are similar in configuration to the second region 52, third region 53, and fourth region 54.

The FPC 43 configured as described above is disposed obliquely at a predetermined angle with respect to the back face 32h in a region superimposed over the package unit 150 when seen in planar view from the image pickup surface 32m.

Also, as shown in Fig. 12, the FPC 43 is disposed, being folded in such a way that the front surface 43i of the FPC 43 will provide mounting surfaces for the electronic components 70 to 72 in three layers and that the electronic components 70 to 72 will be superimposed via the FPC 43.

Furthermore, as shown in Fig. 12, on the FPC 43, the lead wire connecting portions 73 and 74 are disposed in regions different from the regions in which the electronic components 70 to 72 are mounted, i.e., in the top layer. This makes it possible to downsize the mounting space of the FPC 43.

Now, a method for folding the FPC 43 configured as shown in Fig. 13 into a shape shown in Fig. 12 will be described below.

Specifically, the terminals 90 in the fourteenth region 65 are electrically connected to the bonding portion 41 of the solid-state image pickup device chip 32 by soldering or the like. Then, as shown in Fig. 12, first, the rear surface 43t of the FPC 43 in the fourteenth region 65 is affixed to the back face 32h of the solid-state image pickup device chip 32. The tapered portion 131 prevents the adhesive from running out of the narrowed part near the terminals 90 in the fourteenth region 65, as described above.

Next, the fifteenth region 66 is bent obliquely downward in Fig. 12 so as to be at a predetermined angle to the back face 32h of the solid-state image pickup device chip 32. Consequently, the electronic components 70 mounted on the front surface 43i of the FPC 43 in the fifteenth region 66 are oriented obliquely downward in Fig. 12.

Next, the eighteenth region 69 is folded over the fifteenth region 66. Specifically, by bending the deformation portion 167, the eighteenth region 69 is folded in a folding direction R which is a first folding direction so that the rear surface 43t of the FPC 43 in the eighteenth region 69 will face the rear surface 43t of the FPC 43 in the fifteenth region 66. Consequently, the electronic components 71 mounted on the front surface 43i of the FPC 43 in the eighteenth region 69 are oriented obliquely upward in Fig. 12.

Thus, the electronic components 71 are disposed out of direct contact with the electronic components 70. In other words, the electronic components 71 are superimposed over the electronic components 70 via the fifteenth region 66 and eighteenth region 69.

Next, by bending the deformation portion 168, the nineteenth region 81 is bent in the folding direction R so as to be substantially perpendicular to the front surface 43i of the FPC 43 in the fifteenth region 66.

Then, the twentieth region 82 is folded below the fifteenth region 66. Specifically, the twentieth region 82 is folded in the folding direction R via the deformation portion 169 so that the rear surface 43t of the FPC 43 in the twentieth region 82 will face the front surface 43i of the FPC 43 in the fifteenth region 66.

Consequently, the electronic components 72 mounted on the front surface 43i of the FPC 43 in the twentieth region 82 are oriented obliquely downward in Fig. 12. Thus, the electronic components 72 are disposed out of direct contact with the electronic components 70 and 71. In other words, the electronic components 72 are superimposed over the electronic components 70 via the nineteenth region 81 and twentieth region 82. Also, the electronic components 72 are superimposed over the electronic components 71 via the fifteenth region 66, eighteenth region 69, nineteenth region 81, and twentieth region 82.

Next, by bending the deformation portion 165, the sixteenth region 67 is folded over the eighteenth region 69. Specifically, the sixteenth region 67 is folded in the folding direction Q so that the rear surface 43t of the FPC 43 in the sixteenth region 67 will face the front surface 43i of the FPC 43 in the eighteenth region 69.

Consequently, the lead wire connecting portion 73 in the sixteenth region 67 on the front surface 43i of the FPC 43 is oriented obliquely upward in Fig. 12. Thus, the lead wire connecting portion 73 is disposed out of direct contact with the electronic components 71. In other words, the lead wire connecting portion 73 is superimposed over the electronic components 71 via the sixteenth region 67.

Next, by bending the deformation portion 166, the seventeenth region 68 is folded downward in the folding direction Q in Fig. 12. Consequently, the seventeenth region 68 is disposed without jutting out partially from the package unit 150 when seen in planar view from the image pickup surface 32m.

Incidentally, the sixteenth region 67 and seventeenth region 68, which are located in the top layer of the folded FPC 43, are located in regions different from the regions in which the electronic components 70 to 72 are mounted.

Subsequently, to fix the shape of the folded FPC 43 and reinforce the electronic components 70 to 73, an adhesive is applied to a predetermined thickness on the FPC 43.

Finally, the lead wire connecting portions 73 and 74 provided on the front surface 43i of the FPC 43 in the sixteenth region 67 and seventeenth region 68 are electrically connected with multiple lead wires 44 of the signal cable 34, for example, by soldering.

The lead wire connecting portions 73 and 74, which are located in regions different from the regions in which the electronic components 70 to 72 are mounted, specifically, in the top layer of the folded FPC 43, are easily connected with the multiple lead wires 44 of the signal cable 34. This makes it possible to downsize the mounting space of the FPC 43.

Consequently, the FPC 43 is disposed behind the solid-state image pickup chip 43 in the shape and positions as described above.

In this way, according to the present embodiment, after the eighteenth region 69, nineteenth region 81, and twentieth region 82 are folded in the folding direction R, the sixteenth region 67 and seventeenth region 68 are folded over the top layer of the FPC 43.

Thus, the lead wire connecting portions 73 and 74 can be disposed not only in the bottom layer as in the case of the first embodiment described above, but also in the top layer of the FPC 43. Consequently, the lead wires 44 of the signal cable 34 can be connected to the lead wire connecting portions 73 and 74 in the top layer if connecting conditions of the lead wires 44 so require. Other advantages are similar to those of the first embodiment.

Also, according to the first to fifth embodiments described above, the FPC 43 is folded in such a way that the front surface 43i of the FPC 43 will provide mounting surfaces for the electronic components 70 to 72 and 77 in three or four layers, but this is not restrictive and the FPC 43 may be folded into two layers or more than five layers as long as the FPC 43 can be superimposed over the package unit 150 when seen in planar view from the image pickup surface 32m.

Furthermore, according to the first to fifth embodiments described above, it has been assumed that the image pickup apparatus is installed in the distal end portion of the insertion portion of a medical endoscope, but this is not restrictive and the embodiments will provide similar advantages when the image pickup apparatus is installed in the distal end portion of the insertion portion of an industrial endoscope.

## Claims

1. An image pickup apparatus comprising:
a solid-state image pickup device chip;
a flexible printed circuit board connected at one end to the solid-state image pickup device chip; and
a plurality of electronic components mounted on one side of the flexible printed circuit board, wherein
behind a back face opposite an image pickup surface of the solid-state image pickup chip, the flexible printed circuit board is disposed by being folded in such a way that the flexible printed circuit board will provide mounting surfaces for the plurality of electronic components in a plurality of layers and that the electronic components will be superimposed via the flexible printed circuit board.

2. The image pickup apparatus according to claim 1, wherein the flexible printed circuit board provides mounting surfaces for the plurality of electronic components in three or more layers.

3. The image pickup apparatus according to claim 1, wherein:
the solid-state image pickup chip makes up a package unit in conjunction with a connecting portion to which the flexible printed circuit board is connected at the one end and cover glass which protects the image pickup surface by being affixed to the image pickup surface; and
the flexible printed circuit board is disposed in such a position as to be superimposed over the package unit when seen in planar view from the image pickup surface.

4. The image pickup apparatus according to claim 1, wherein the flexible printed circuit board is disposed by being inclined at a predetermined angle with respect to the back face of the solid-state image pickup chip.

5. The image pickup apparatus according to claim 1, wherein the electronic component smaller than a predetermined size is disposed in a region surrounded by the plurality of electronic components equal to or larger than the predetermined size when seen in planar view.

6. The image pickup apparatus according to claim 1, wherein the electronic component smaller than a predetermined size is disposed in a region sandwiched by the plurality of electronic components equal to or larger than the predetermined size when seen in planar view.

7. The image pickup apparatus according to claim 6, wherein the plurality of electronic components equal to or larger than the predetermined size are disposed in such a way as to sandwich the electronic component smaller than the predetermined size in a direction orthogonal to a folding direction of the flexible printed circuit board, when seen in planar view.

8. The image pickup apparatus according to claim 1, wherein a mounting strength enhancement portion is formed in at least part of ends of the flexible printed circuit board in a direction perpendicular to a folding direction of the flexible printed circuit board to enhance mounting strength of the electronic components on the flexible printed circuit board in the folding direction of the flexible printed circuit board.

9. The image pickup apparatus according to claim 8, wherein the electronic component smaller than the predetermined size is disposed near the mounting strength enhancement portion.

10. The image pickup apparatus according to claim 1, wherein part of the plurality of electronic components is mounted, on one side of the flexible printed circuit board, in a region folded in a second folding direction different from a first folding direction which is the folding direction.

11. The image pickup apparatus according to claim 1, wherein a lead wire connecting portion is installed at the other end on either one of the one side of the flexible printed circuit board and the other side opposite the one side in order to be connected with lead wires of a signal cable which transmits an electrical signal of an image picked up on the image pickup surface of the solid-state image pickup chip to an external apparatus.

12. The image pickup apparatus according to claim 11, wherein on the folded flexible printed circuit board, the lead wire connecting portion is located in a region separate from a region in which the plurality of electronic components are installed.

13. The image pickup apparatus according to claim 1, wherein the flexible printed circuit board has the shape fixed by an adhesive in a folded state.

14. An endoscope which includes an image pickup apparatus, the image pickup apparatus comprising:
a solid-state image pickup device chip;
a flexible printed circuit board connected at one end to the solid-state image pickup device chip; and
a plurality of electronic components mounted on one side of the flexible printed circuit board, wherein
behind a back face opposite an image pickup surface of the solid-state image pickup chip, the flexible printed circuit board is disposed by being folded in such a way that the flexible printed circuit board will provide mounting surfaces for the plurality of electronic components in a plurality of layers and that the electronic components will be superimposed via the flexible printed circuit board.
